# EUROPEAN PATENT SPECIFICATION

(11) **EP 4 406 559 B1**
(45) Date of publication and mention of the grant of the patent: **19.11.2025**
(21) Application number: 24153636.6
(22) Date of filing: 24.01.2024
(51) Int. Cl.: A61L 2/04, A61L 2/24, B08B 3/12

(54) **PLANT AND CORRESPONDING METHOD FOR TREATING OBJECTS**
ANLAGE UND ENTSPRECHENDES VERFAHREN ZUR BEHANDLUNG VON GEGENSTÄNDEN
INSTALLATION ET PROCÉDÉ CORRESPONDANT POUR TRAITER DES OBJETS

(30) Priority: 24.01.2023 IT 202300000960
(43) Date of publication of application: 31.07.2024
(73) Proprietor: Steelco S.p.A., 31039 Riese Pio X (IT)
(72) Inventor: Caprara, Alessandro, 37063 Isola Della Scala (VR) (IT); Gremese, Mattia, 33078 San Vito al Tagliamento (PN) (IT)
(74) Representative: Petraz, Gilberto Luigi

(56) References cited:
- EP-A2- 1 787 731
- US-A1- 2010 206 335
- US-A1- 2018 318 458

## Description

### FIELD OF THE INVENTION

The present invention concerns a plant and a method for treating objects, such as medical instruments for hospital departments, operating theatres, possibly laboratories, and the pharmaceutical industry. In particular, the treatment plant comprises one or more pre-treatment stations and one or more machines for treating the aforementioned objects, which are disposed in object-holding baskets moved by means of an unmanned shuttle.

### BACKGROUND OF THE INVENTION

Hospital facilities are known to be provided with a plant for treating objects, as specified above.

These plants can be part of treatment centers for carrying out the pre-wash, wash, thermal disinfection and sterilization of the aforementioned objects, which are divided into several areas, isolated from each other for hygienic reasons and called, respectively, "septic" or "dirty" area, or reception, "clean" area and "sterile" area.

In particular, baskets arrive in the first area which contain the dirty objects to be subjected to the various treatments, including a pre-wash with cold water only, a possible wash in an ultrasonic bath, a wash with hot water and possible detergents, the necessary rinses, a thermal disinfection and a final drying.

Usually, the pre-wash is carried out by means of one or more pre-washing stations, or machines, for example two or three, disposed aligned according to a certain axis of alignment, so as to operate in series with each other.

The cycle that provides the wash, rinse, thermal disinfection and drying, on the other hand, is carried out in an array of washing and thermal disinfection machines, for example five or six, according to the productivity required, which are aligned according to a respective axis of alignment, so as to operate in parallel with each other.

Each washing and thermal disinfection machine has a washing chamber with an aperture facing the septic area and an opposing aperture facing the clean area; therefore, the baskets with the pre-washed objects are fed into a washing and thermal disinfection machine in a direction orthogonal to its axis of alignment with the other machines.

In particular, the baskets are picked up at the exit of the last pre-washing machine and fed to the washing and thermal disinfection machine available at that time.

Normally, this feed is carried out by means of one or more autonomous shuttles that receive the baskets containing the objects to be washed from the outlet of the pre-washing stations and transport them in correspondence with the inlet of one of the washing machines, selected on each occasion as a function of the washing program set, or depending on availability.

After having been thermally disinfected and dried the objects pass into the clean area, where they are packaged in specific packaging stations and, from here, are fed by means of other autonomous shuttles to an array of sterilization machines, generally autoclaves, which provide to sterilize them, operating in parallel, and which are typically aligned in a direction parallel to the pre-washing and thermal disinfection array.

The sterilization machines also have an aperture facing the clean area and an opposing aperture facing the sterile area and, once the sterilization has been carried out, the objects pass to the next sterile area where they are stored or once again sent to be used in the operating theatre.

There are example embodiments of such treatment plants and/or centers in European patent applications EP-A-1.787.662 and EP-A-1.787.731, filed in the name of the Applicant. Further examples can be found in US 2018/318458 which discloses a plant for treating objects, comprising pick-up benches, treatment machines, and a shuttle for moving baskets between stations; US 2010/206335 which discloses a washing plant with a shuttle and sequential processing steps; and EP 1 787 731 which discloses a treatment plant with controllers and sensors.

In addition, treatment plants are known in which, for the correct and optimized movement of the shuttles, both the latter and also each pre-washing station and washing and thermal disinfection machine are provided with respective control devices, even of different types, communicating with each other directly, or indirectly by means of a central control unit connected to them.

However, the large number of control devices, even of different types, can lead to the disadvantage that problems of interaction and/or software and/or hardware connection can occur between the shuttles, the pre-washing stations and washing and thermal disinfection machines, for example related to the proportional number of communications that have to be generated.

In addition, in such known plants the movement of the autonomous shuttle toward the pick-up bench of the pre-washing machines is started through an input from an operator, therefore not in an automated manner. Therefore, if the operator is not careful, or is unable to start such movement, slowdowns or interruption of the cycle of treatment operations can occur.

There is therefore the need to perfect a plant and method for treating objects that can overcome at least one of the disadvantages of the state of the art.

One purpose of the present invention is to provide a plant and perfect a method for treating objects which are highly efficient and automated.

Another purpose of the present invention is to provide a plant and perfect a method for treating objects which allow to reduce the operating times of the treatment cycle of the aforementioned objects, and which also allow for significant economic savings.

Another purpose of the present invention is to provide a plant and perfect a method for treating objects in which it is possible to track both the objects to be treated and also the baskets in which they are disposed.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes and to resolve the technical problem disclosed above in a new and original way, also achieving considerable advantages compared to the state of the prior art, a plant according to the present invention for treating objects comprises a plurality of pre-treatment stations, a plurality of pick-up benches for picking up baskets containing already pre-treated objects, associated with the pre-treatment stations, a plurality of treatment machines for treating the pre-treated objects, and at least one mobile unmanned shuttle provided on board with a first control device for receiving and executing movement information relating to least one path between the pick-up benches and respective inlet apertures of the treatment machines.

In accordance with one aspect of the present invention, the plant comprises a supervision system provided with visual acquisition means connected to a central control unit which is communicating at least with the first control device.

In particular, the visual acquisition means are configured to frame at least the pick-up benches on which baskets of pre-treated objects are provided, and to acquire visual information about a pick-up condition thereof.

The central control unit is configured to receive and process both the visual information and also information on the availability status of the treatment machines, to generate the movement information in order to command the movement of the at least one shuttle along the at least one path, in order to pick-up the basket in the pick-up condition and transport it to a respective treatment machine. In order to communicate with at least said central control unit, said first control device is provided with a communication interface and said treatment machines each comprise second control devices provided with respective communication interfaces, wherein visual indication means are associated with said pick-up benches and/or with each basket, to indicate the presence of a basket, loaded on the respective pick-up bench, in said pick-up condition, and wherein said visual acquisition means are also able to detect the indication of said visual indication means to obtain information usable for said central control unit.

In accordance with another aspect of the present invention, the acquisition means - which comprise at least one video camera - are configured to also frame the treatment machines in order to acquire information on the availability status thereof, usable for the central control unit.

In accordance with another aspect of the present invention, the indication means are status light elements associated with the pick-up benches and configured to selectively emit a light radiation able to indicate the pick-up condition of a corresponding basket.

In accordance with another aspect of the present invention, the indication means are coding elements which are associated with respective baskets, and which are readable and/or detectable by the visual acquisition means in order to give an indication of the pick-up condition of a corresponding basket.

Other embodiments also comprise a method for treating objects by means of said plant, comprising a pre-treatment step, in which the objects are pre-treated in one or more of the pre-treatment stations, a positioning step, in which the pre-treated objects are positioned in respective baskets suitably disposed on the pick-up benches, a movement step, in which at least one shuttle, by means of the first control device which executes received movement information relating to the path, moves between the pick-up benches and respective inlet apertures of the treatment machines, and a treatment step, in which the pre-treated objects disposed in the baskets are treated in one of the treatment machines.

In accordance with another aspect of the present invention, the method comprises a control and supervision step in which, in an acquisition sub-step, the visual acquisition means frame at least the pick-up benches to acquire visual information on a pick-up condition of a basket provided with pre-treated objects which is positioned on one of the pick-up benches in a certain pick-up position, and in which, in a processing sub-step, the central control unit receives and processes both the visual information and also information on the availability status of the treatment machines, to generate the movement information in order to command the movement of the at least one shuttle along the at least one path, to pick up the basket in the pick-up condition and transport it to a respective treatment machine.

In accordance with another aspect of the present invention, in the acquisition sub-step the visual acquisition means also frame the treatment machines to acquire information on the availability status thereof, making it usable for the central control unit.

In accordance with another aspect of the present invention, the control and supervision step comprises, in addition or alternatively, a transmission sub-step in which the second control devices transmit information on the availability status relating to the treatment machines to the central control unit.

In accordance with another aspect of the present invention, in the acquisition sub-step, the visual acquisition means detect and acquire information, usable for the central control unit, which is obtained, or generated, by visual indication means associated with the pick-up benches and/or with respective baskets, wherein the information indicates the presence of a basket in a pick-up condition.

Other embodiments also concern a treatment center for carrying out the complete treatment of pre-washing, washing, thermal disinfection and sterilization of objects, which comprises at least a first septic area, a second clean area, and a third sterile area, which are separated from each other by separation means.

In particular, the first area is defined by a plant according to the present invention, wherein the pre-treatment stations are stations for pre-washing the objects and the treatment machines are machines for washing and thermally disinfecting the already pre-washed objects, and wherein the second area is defined by another plant according to the present invention, wherein the pre-treatment stations are stations for packaging the washed and thermo-disinfected objects and the treatment machines are machines for sterilizing the objects, in order to prepare them for a subsequent storage in the third area.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic plan representation of a plant for treating objects, according to the present invention;
- fig. 2 is a schematic plan representation of a treatment center in which there are two plants for treating objects, according to the present invention;
- fig. 3 is a schematic and simplified three-dimensional representation of a part of the plant of fig. 1 according to a first variant;
- fig. 4 is a schematic and simplified three-dimensional representation of a part of the plant of fig. 1 according to a second variant;
- fig. 5 is a schematic and simplified three-dimensional representation of a part of the plant of fig. 1 according to a third variant.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVENTION

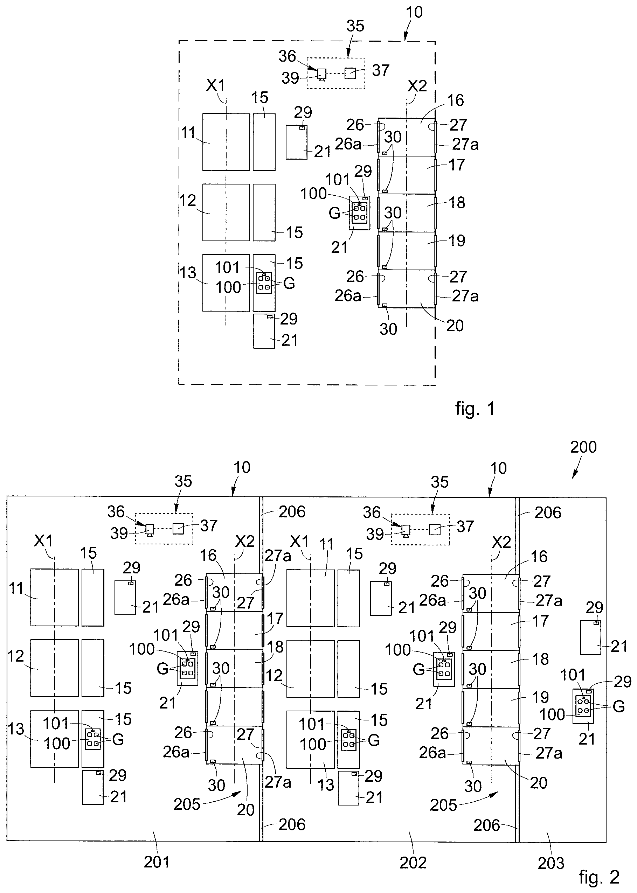

With reference to fig. 1, a plant 10 according to the present invention is suitable and usable to carry out the treatment of objects G, such as instruments coming from hospital wards, operating theatres, laboratories and the pharmaceutical industry, generally contained and transported in special baskets 100.

The objects G can be fed to the plant 10 manually by an operator, semi-automatically, or automatically by means of suitable feed means, not shown in the drawings.

The plant 10 comprises a plurality of pre-treatment stations 11, 12, 13 for pretreating the objects G, a plurality of pick-up benches 15 associated with the pre-treatment stations 11, 12, 13 and on which baskets 100 able to contain the already pre-treated objects G can be positioned, and a plurality of treatment machines 16, 17, 18, 19, 20 for treating the pre-treated objects G contained in the baskets 100.

By way of example, the objects G can be disposed on trays, or containers, not shown in the drawings. In addition, the baskets 100 are able to contain the objects G disposed on such trays, or containers, and can be of different types depending on requirements or on the type of treatment machines 16, 17, 18, 19, 20 in which they are inserted.

The plant 10 comprises at least one unmanned, that is, autonomous, shuttle 21 mobile along at least one path between the pick-up benches 15 and the treatment machines 16, 17, 18, 19, 20, as will be described in detail below. Preferably, the plant 10 comprises a plurality of shuttles 21 mobile between the pick-up benches 15 and the treatment machines 16, 17, 18, 19, 20.

It should be noted that each shuttle 21 can be mobile in contact with the floor of the plant 10 along the aforementioned path, for example by using means on wheels, or suchlike. Alternatively, each shuttle 21 can be mobile, along the aforementioned path, without contacting the floor of the plant 10, that is, being raised therefrom by a certain distance.

According to possible embodiments, shown in figs. 1 and 2, the pre-treatment stations 11, 12, 13 can be aligned along a respective first axis of alignment X1 and can operate in series. It should be noted that by the expression "operate in series" we mean that the objects G that exit from one pre-treatment station 11, 12, 13 face the next pre-treatment station in order to be possibly introduced therein for another pre-treatment.

According to other possible embodiments, the pre-treatment stations 11, 12, 13 can be disposed in a different manner, therefore not aligned with each other, or they may not operate in series.

Furthermore, each pre-treatment station 11, 12, 13 can be provided with two or more modules, not shown in the drawings, which allow to perform the pre-treatment operations in series.

The pick-up benches 15 are associated with the pre-treatment stations 11, 12, 13 and are external to, or preferably separate from, them; preferably, they can be disposed to the side of the latter and facing the treatment machines 16, 17, 18, 19, 20.

It should be noted that the configuration of the pick-up benches 15 can be different from the one shown, solely by way of example, in the drawings, that is, that with each pre-treatment station 11, 12, 13 there is associated a pick-up bench 15. In particular, one pick-up bench 15 can be associated with two or more pick-up stations 11, 12, 13, also in relation to their number.

In particular, the pick-up benches 15 are provided with a rest plane 22 on which the baskets 100, with the pre-treated objects G inside them, can be positioned. In the example given here, each rest plane 22 can be defined by a plurality of feed rollers 23 to make the baskets 100 advance and position them, one at a time, in a certain pick-up position, in order to be picked up by a respective shuttle 21, which is located, or positioned, at a certain pick-up point next to the corresponding pick-up bench 15.

For example, the pick-up point can be a free area in front of the corresponding pick-up bench 15, able to allow appropriate approaching and positioning maneuvers of the corresponding shuttle 21.

According to possible embodiments, shown in figs. 1 and 2, the treatment machines 16, 17, 18, 19, 20 are aligned along a second axis of alignment X2 and operate in parallel.

Each treatment machine 16, 17, 18, 19, 20 is provided with a tunnel treatment chamber 25 having an inlet aperture 26 and an opposing outlet aperture 27. Both the inlet aperture 26 and also the outlet aperture 27 are selectively closable by means of respective inlet doors 26a and outlet doors 27a, preferably of the shutter type. Specifically, the inlet apertures 26 face the pick-up benches 15.

We must clarify that by the expression "operate in parallel" we mean that the treatment machines 16, 17, 18, 19, 20 are side by side to each other and have their respective inlet apertures 26 disposed in parallel to each other and on the same plane, to facilitate the insertion of the baskets 100 containing the pre-treated objects G, which are transported by the shuttles 21.

Specifically, each shuttle 21 is mobile along a path between the pick-up benches 15 and respective inlet apertures 26 of the treatment machines 16, 17, 18, 19, 20.

Each shuttle 21 comprises movement means for moving it along the aforementioned path and at least one housing plane 28 for the baskets 100 containing the objects G.

A possible example of a shuttle 21 is described in European patent application EP-A-1.787.731 in the name of the Applicant.

In addition, each shuttle 21 is provided on board with a first control device 29 configured to receive and execute movement information relating to the aforementioned path.

We must clarify that the treatment machines 16, 17, 18, 19, 20 also each comprise respective second control devices 30 for controlling and acquiring information on the availability status and/or operating status thereof.

According to some embodiments, the pre-treatment stations 11, 12, 13 are stations for pre-washing the dirty objects G to prepare them for the subsequent washing and thermal disinfection treatment. For example, the pre-wash can be obtained by means of a cold pre-wash, wash with possible chemical detergents and/or wash in an ultrasonic bath. In these embodiments, the treatment machines 16, 17, 18, 19, 20 are machines for washing and thermally disinfecting the pre-washed objects G to prepare them for a subsequent treatment, for example sterilization.

According to other embodiments, the pre-treatment stations 11, 12, 13 are stations for packaging the washed and thermally disinfected objects G, to arrange them in packages for the subsequent sterilization treatment. In these embodiments, the treatment machines 16, 17, 18, 19, 20 are machines for sterilizing the packaged objects G to prepare them for a subsequent storage.

The plant 10 comprises a supervision system 35 provided with visual acquisition means 36 connected to a central control unit 37. Specifically, the supervision system 35 is external to the pre-treatment stations 11, 12, 13 and the treatment machines 16, 17, 18, 19, 20.

The visual acquisition means 36 are configured to frame at least the pick-up benches 15 in order to acquire visual information about a pick-up condition of a basket 100 loaded with pre-treated objects G and disposed on one of the pick-up benches 15.

According to possible embodiments, the visual acquisition means 36 comprise at least one video camera 39 positioned above the pre-treatment machines 11, 12, 13 to frame at least the area of the plant 10 in which the pick-up benches 15 are present.

In addition, the video camera 39 can be disposed in such a way as to be able to also frame the area in which the inlet apertures 26 of the treatment machines 16, 17, 18, 19, 20 are present, to obtain visual information on the availability and/or operating status of the latter. For example, the availability status can be visually identified when the inlet door 26a is in an open position, so as to make the inlet aperture 26 accessible.

According to other possible embodiments, not shown in the drawings, the visual acquisition means 36 comprise one or more video cameras 39 disposed for example at least above the pick-up benches 15 and the treatment machines 16, 17, 18, 19, 20.

The central control unit 37 is communicating at least with the first control device 29. In particular, the first control device 29 is provided with a communication interface capable of communicating with the central control unit 37, for example in wireless mode.

The central control unit 37 is configured to receive and process both the visual information and also the information on the availability status of the treatment machines 16, 17, 18, 19, 20 to generate the aforementioned movement information in order to command the movement of the shuttles 21 to pick up a certain basket 100 in the pick-up condition, and transport it to a respective treatment machine 16, 17, 18, 19, 20. In particular, the central control unit 37 is able to process the visual information and/or the status information received from the visual acquisition means 36 and/or from the second control devices 30 in order to generate movement information to be communicated to the first control devices 29 present on each shuttle 21.

We must clarify that by the expression "pick-up condition" we mean that a basket 100 is loaded with the pre-treated objects G and is correctly positioned on a pick-up bench 15 ready to be picked up by means of a respective shuttle 21.

According to some embodiments, the central control unit 37 is also communicating with the second control devices 30 of the treatment machines 16, 17, 18, 19, 20. Specifically, the second control devices 30 can also be provided with a communication interface for communicating with the central control unit 37 in order to transmit status information relating to the availability and/or operation of the treatment machines 16, 17, 18, 19, 20.

In accordance with other embodiments, in the plant 10 there are visual indication means 40 capable of indicating the presence of a basket 100 loaded on the respective pick-up bench 15 in a correct pick-up condition.

This is particularly useful for a greater usability of the pick-up bench 15 by an operator, who can easily and quickly be updated on the operating or availability status of a corresponding pick-up bench 15.

Advantageously, the acquisition means 36 are also capable of detecting the indication generated by the visual indication means 40 to obtain information usable for the central control unit 37. Specifically, the visual indication means 40 can be associated with the pick-up benches 15 and/or with respective baskets 100.

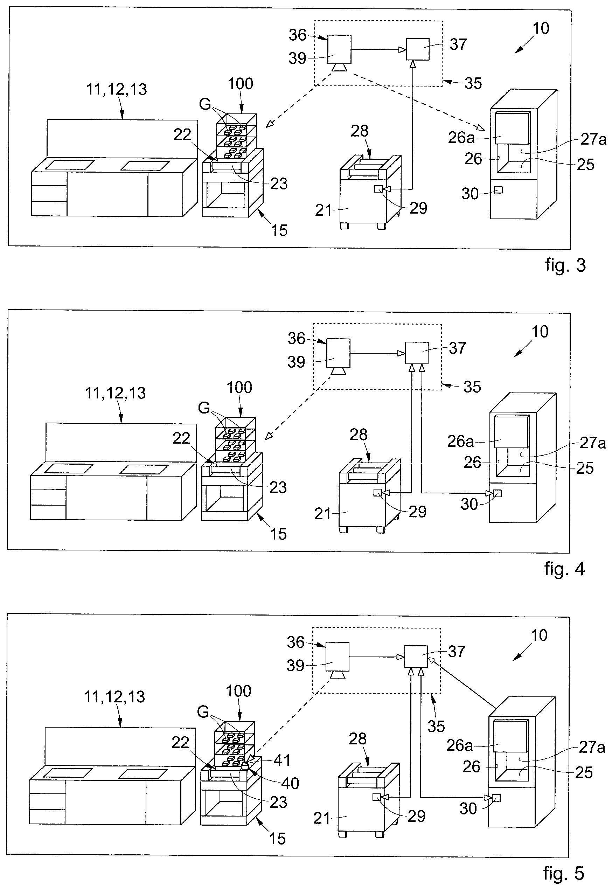

As shown in fig. 5, the visual indication means 40 can be status light elements 41 associated with the pick-up benches 15 and configured to selectively emit a light radiation able to indicate that the basket 100 is in a correct pick-up condition. Therefore, in this case, the video camera 39 is disposed in such a way that it can detect the light radiation emitted by the respective status light element 41 and acquire specific visual information to be made usable for the central control unit 37, so that the latter can then process it in order to obtain movement information for a shuttle 21.

According to possible embodiments, the light radiation can be activated automatically, or manually by an operator.

According to other embodiments, not shown in the drawings, the visual indication means 40 can also be physical signaling elements associated with the pick-up benches 15 and mechanically drivable, for example batons or suchlike, possibly colored, capable of being selectively activated to signal to at least one video camera 39 that the basket 100 is in a correct pick-up condition.

According to other possible embodiments, the visual indication means 40 can be coding elements 101 associated with each basket 100. Each coding element 101 is preferably a graphic code identifying the same basket 100 and is readable by the visual acquisition means 36 to give usable information to the central control unit 37. It should be noted that in this case the visual acquisition means 36 are configured to frame the pick-up benches 15, and in particular the corresponding coding elements 101 of the baskets 100.

The coding element 101, that is, the graphic identification code, can give information on the status of the respective basket 100, in particular if it is in the respective pick-up condition, or information relating to its position. For example, the coding element 101 can be a barcode, in particular a linear barcode or a 2D barcode, such as a QR code.

This achieves the advantage of being able to track the movement of the baskets 100 and of the objects G inside the plant 10.

In accordance with possible preferred embodiments, a treatment center 200 for carrying out the complete treatment of pre-washing, washing, thermal disinfection and sterilization of objects G comprises at least two treatment plants 10 according to the present invention.

In particular, the treatment center 200 comprises a first area 201, or "septic" or "dirty" area, a second area 202, or "clean" area, and a third area 203, or "sterile" area, which are separated from each other by means of separation means 205.

The first area 201 can be defined by a plant 10 in which the pre-treatment stations 11, 12, 13 are stations for pre-washing the dirty objects G and the treatment machines 16, 17, 18, 19, 20 are machines for washing and thermally disinfecting the pre-washed objects G.

The second area 202 can be defined by another plant 10 in which the pre-treatment stations 11, 12, 13 are stations for packaging the washed and thermally disinfected objects G, and the treatment machines 16, 17, 18, 19, 20 are machines for sterilizing the packaged objects G, in order to prepare them for a subsequent storage.

In the third area 203 the objects G arrive sterilized, preferably in packets, and can be stored for subsequent use. By way of example, the objects G can be also disposed on trays, or containers.

According to possible embodiments, there can be a supervision system 35 also inside the third area 203 and one or more shuttles 21 can be present to pick-up the baskets 100 from the treatment machines 16, 17, 18, 19, 20 and transport them into one or more storage or sorting zones, in which storage members are present, not shown in the drawings.

The separation means 205 of each area 201, 202, 203 can be defined by at least two opposing separation walls 206 and by the overall dimensions of the treatment machines 16, 17, 18, 19, 20 associated with the separation walls 206. Specifically, the treatment machines 16, 17, 18, 19, 20 substantially constitute a communication interface between the first area 201 and the second area 202, and between the second area 202 and the third area 203.

The operation of the plant 10 described heretofore, which corresponds to the method according to the present invention, comprises the following steps.

An initial preparation step, in which the objects G to be treated, possibly disposed in trays, or containers, are arranged in a known manner in correspondence with the pre-treatment stations 11, 12, 13 to be pre-treated.

A subsequent pre-treatment step, in which the objects G present in the baskets 100 are pre-treated in one or more of the pre-treatment stations 11, 12, 13.

Subsequently, in a positioning step, the pre-treated objects G are suitably positioned in baskets 100 disposed on one or more pick-up benches 15. These baskets 100, once properly loaded, are suitably moved into a certain pick-up position on the corresponding pick-up bench 15, thus defining a corresponding pick-up condition.

Then, in a control and supervision step, the supervision system 35 carries out the control of the operating, or status, conditions of the pick-up benches 15 and of the treatment machines 16, 17, 18, 19, 20.

The control and supervision step comprises an acquisition sub-step, in which the visual acquisition means 36 frame at least the pick-up benches 15 to acquire visual information on the pick-up condition of a basket 100 provided with pre-treated objects G, present on one of the pick-up benches 15. Specifically, in this step the video camera 39 frames the correct positioning of the basket 100 provided with pre-treated objects G on the rest plane 22 of the pick-up bench 15.

In the acquisition sub-step, the visual acquisition means 36 detect and acquire information, usable for the central control unit 37, which is detectable by the visual indication means 40, wherein said information indicates the presence of a basket 100 in a pick-up condition.

According to some embodiments, this information is detectable by the coding element 101 of a basket 100 which is in the pick-up condition.

According to a first operating variant, in the visual acquisition sub-step the visual acquisition means 36 also frame the treatment machines 16, 17, 18, 19, 20 to acquire information on the availability and/or operating status of the treatment machines 16, 17, 18, 19, 20. For example, in this step the video camera 39 frames whether the inlet door 26a is open and/or whether the inlet aperture 26 is accessible.

Then, the visual and/or status information is transmitted to the central control unit 37 which, in a processing sub-step, processes the visual information to obtain movement information to be communicated to a respective first control device 29 of an available shuttle 21, for the corresponding movement.

In particular, in the first operating variant, the central control unit 37 processes both the visual information relating to the pick-up benches 15 and also the availability status information relating to the treatment machines 16, 17, 18, 19, 20 acquired by means of the video camera 39 to transmit the movement information to an available shuttle 21.

In a second and third operating variant, the control and supervision step also comprises a transmission sub-step, in which the second control devices 30 transmit information on the availability and/or operating status relating to the treatment machines 16, 17, 18, 19, 20 because the visual acquisition means 36, that is, the video camera 39, acquire visual information only of the pick-up benches 15.

In this operating situation, in the processing sub-step the central control unit 37 processes the visual information relating to the pick-up benches 15 and the status information relating to the treatment machines 16, 17, 18, 19, 20 in order to transmit suitable movement information to a respective first control device 29 of an available shuttle 21, for the corresponding movement.

In accordance with the third variant, during the acquisition sub-step the visual acquisition means 36 frame the pick-up benches 15, in particular the visual indication means 40 to acquire visual information on a pick-up condition of a basket 100 loaded with already pre-treated objects G.

Specifically, according to some embodiments, the video camera 39 frames the status light element 41 which in that moment signals a pick-up condition of a corresponding basket 100 and acquires visual information usable for the central processing unit 37 for the next processing sub-step.

Then, once the movement information has been communicated, a movement step begins, in which by means of the first control device 29 at least one shuttle 21 executes received movement information, relating to the path, so that it moves between the pick-up benches 15, in correspondence with certain pick-up points close to the latter and respective inlet apertures 26 of the treatment machines 16, 17, 18, 19, 20.

In particular, during the movement step, one of the shuttles 21 is disposed in correspondence with one of the pick-up benches 15 to receive a basket 100 which is in the pick-up condition, and which is subsequently disposed on the housing plane 28 of the same shuttle 21. Subsequently, this shuttle 21 transports the basket 100 in correspondence with an available treatment machine 16, 17, 18, 19, 20 to position it inside the corresponding treatment chamber 25.

A treatment step then begins, in which the objects G contained in a corresponding basket 100 are treated in one of the treatment machines 16, 17, 18, 19, 20.

Considering the operation of the treatment center 200, in the first of the two plants 10, during the pre-treatment step the dirty objects G present in the baskets 100 are pre-washed, and in the treatment step the pre-washed objects G are washed and thermally disinfected. Instead, in the second of the two plants 10, during the pre-treatment step the washed and thermally disinfected objects G are packaged, and in the treatment step the washed, thermally disinfected and packaged objects G are sterilized.

We must clarify that the movement of the objects from the outlet apertures 27 of the treatment machines 16, 17, 18, 19, 20 of the first of the two plants 10 to the pre-treatment stations 11, 12, 13 of the second of the two plants 10 can occur in any suitable manner whatsoever. For example, it can be done manually by means of an operator, semi-automatically or automatically with one or more shuttles 21.

It is also clear that, although the present invention has been described with reference to some specific examples, a person of skill in the art will be able to achieve other equivalent forms of plants and methods for treating objects, having the characteristics as set forth in the claims and hence all coming within the field of protection defined thereby.

In the following claims, the sole purpose of the references in brackets is to facilitate their reading and they must not be considered as restrictive factors with regard to the field of protection defined by the claims.

## Claims

1. Plant (10) for treating objects (G) comprising a plurality of pre-treatment stations (11, 12, 13), a plurality of pick-up benches (15) configured for picking up baskets (100) containing pre-treated objects (G) and positioned near said pre-treatment stations (11, 12, 13), a plurality of treatment machines (16, 17, 18, 19, 20), and at least one mobile unmanned shuttle (21) provided on board with a first control device (29) for receiving and executing movement information relating to least one path between said pick-up benches (15) and respective inlet apertures (26) of said treatment machines (16, 17, 18, 19, 20), **characterized in that** it comprises a supervision system (35) provided with visual acquisition means (36) connected to a central control unit (37) which is communicating at least with said first control device (29), said visual acquisition means (36) being configured to frame at least said pick-up benches (15) in order to acquire visual information about a pick-up condition of a basket (100) provided with pre-treated objects (G) which is present on one of said pick-up benches (15), wherein said central control unit (37) is configured to receive and process both said visual information and also information on the availability status of said treatment machines (16, 17, 18, 19, 20), to generate said movement information in order to command said movement of said at least one shuttle (21), wherein in order to communicate with at least said central control unit (37), said first control device (29) is provided with a communication interface and said treatment machines (16, 17, 18, 19, 20) each comprise second control devices (30) provided with respective communication interfaces, wherein visual indication means (40) are associated with said pick-up benches (15) and/or with each basket (100), to indicate the presence of a basket (100), loaded on the respective pick-up bench (15), in said pick-up condition, and wherein said visual acquisition means (36) are also able to detect the indication of said visual indication means (40) to obtain information usable for said central control unit (37).

2. Treatment plant (10) as in claim 1, **characterized in that** said visual acquisition means (36), comprising at least one video camera (39), are configured to also frame said treatment machines (16, 17, 18, 19, 20) in order to acquire information on the availability status thereof usable for said central control unit (37).

3. Treatment plant (10) as in claim 1, **characterized in that** said indication means (40) are status light elements (41) associated with said pick-up benches (15) and configured to selectively emit a light radiation able to indicate said pick-up condition of a corresponding basket (100).

4. Treatment plant (10) as in claim 1, **characterized in that** said indication means (40) are coding elements (101) which are associated with respective baskets (100), and which are readable and/or detectable by said visual acquisition means (36) in order to give an indication of said pick-up condition of a corresponding basket (100).

5. Method for treating objects (G) by means of a plant (10) as in claims from 1 to 4, comprising a pre-treatment step, in which said objects (G) are pre-treated in one or more of said pre-treatment stations (11, 12, 13), a positioning step, in which said pre-treated objects (G) are positioned in respective baskets (100) suitably disposed on said pick-up benches (15), a movement step, in which at least one shuttle (21), by means of said first control device (29) which executes received movement information relating to said path, moves between said pick-up benches (15) and respective inlet apertures (26) of said treatment machines (16, 17, 18, 19, 20), and a treatment step, in which said objects (G) are treated in one of said treatment machines (16, 17, 18, 19, 20), **characterized in that** it comprises a control and supervision step in which, in an acquisition sub-step, said visual acquisition means (36) frame at least said pick-up benches (15) to acquire visual information on a pick-up condition of said basket (100) provided with pre-treated objects (G) which is positioned on said pick-up benches (15) in a certain pick-up position, and in which, in a processing sub-step, said central control unit (37) receives and processes both said visual information and also information on the availability status of said treatment machines (16, 17, 18, 19, 20), to generate said movement information in order to command said movement of said at least one shuttle (21), wherein in order to communicate with at least said central control unit (37), said first control device (29) is provided with a communication interface and said treatment machines (16, 17, 18, 19, 20) each comprise second control devices (30) provided with respective communication interfaces, wherein visual indication means (40) are associated with said pick-up benches (15) and/or with each basket (100), to indicate the presence of a basket (100), loaded on the respective pick-up bench (15), in said pick-up condition, and wherein said visual acquisition means (36) are also able to detect the indication of said visual indication means (40) to obtain information usable for said central control unit (37).

6. Method as in claim 5, **characterized in that** in said acquisition sub-step said visual acquisition means (36) also frame said treatment machines (16, 17, 18, 19, 20) to acquire information on the availability status thereof, making it usable for said central control unit (37).

7. Treatment center (200) for carrying out the complete treatment of pre-washing, washing, thermal disinfection and sterilization of objects (G), which comprises at least a first septic area (201), a second clean area (202), and a third sterile area (203), which are separated from each other by separation means (205), wherein said first area (201) is defined by a plant (10) as in claims from 1 to 4, wherein said pre-treatment stations (11, 12, 13) are stations for pre-washing said objects (G) and said treatment machines (16, 17, 18, 19, 20) are machines for washing and thermally disinfecting said already pre-washed objects (G), and wherein said second area (202) is defined by another plant (10) as in claims from 1 to 4, wherein said pre-treatment stations (11, 12, 13) are stations for packaging said washed and thermo-disinfected objects (G) and said treatment machines (16, 17, 18, 19, 20) are machines for sterilizing said objects (G), in order to prepare them for a subsequent storage in said third area (203).

## Patentansprüche

1. Anlage (10) zur Behandlung von Gegenständen (G), aufweisend eine Mehrzahl von Vorbehandlungsstationen (11, 12, 13), eine Mehrzahl von Aufnahmebänken (15), die zum Aufnehmen von Körben (100), die vorbehandelte Gegenstände (G) enthalten, konfiguriert sind und die in der Nähe der Vorbehandlungsstationen (11, 12, 13) angeordnet sind, eine Mehrzahl von Behandlungsmaschinen (16, 17, 18, 19, 20) und mindestens ein mobiles unbemanntes Shuttle (21), das an Bord mit einer ersten Steuervorrichtung (29) versehen ist zum Empfangen und Ausführen von Bewegungsinformationen in Bezug auf mindestens einen Weg zwischen den Aufnahmebänken (15) und jeweiligen Einlassöffnungen (26) der Behandlungsmaschinen (16, 17, 18, 19, 20), **dadurch gekennzeichnet, dass** sie ein Überwachungssystem (35) aufweist, das mit visuellen Erfassungsmitteln (36) versehen ist, die mit einer zentralen Steuereinheit (37) verbunden sind, die zumindest mit der ersten Steuervorrichtung (29) kommuniziert, wobei die visuellen Erfassungsmittel (36) so konfiguriert sind, dass sie zumindest die Aufnahmebänke (15) rahmenerfassen, um visuelle Informationen über einen Aufnahmezustand eines mit vorbehandelten Gegenständen (G) versehenen Korbs (100) zu erlangen, der auf einer der Aufnahmebänke (15) vorliegt, wobei die zentrale Steuereinheit (37) konfiguriert ist, um sowohl die visuellen Informationen als auch Informationen über den Verfügbarkeitsstatus der Behandlungsmaschinen (16, 17, 18, 19, 20) zu empfangen und zu verarbeiten, um die Bewegungsinformationen zu erzeugen, um die Bewegung des mindestens einen Shuttles (21) zu steuern, wobei die erste Steuervorrichtung (29) zur Kommunikation mit mindestens der zentralen Steuereinheit (37) mit einer Kommunikationsschnittstelle versehen ist und die Bearbeitungsmaschinen (16, 17, 18, 19, 20) jeweils zweite Steuervorrichtungen (30) aufweisen, die mit jeweiligen Kommunikationsschnittstellen versehen sind, wobei visuelle Anzeigemittel (40) mit den Aufnahmebänken (15) und/oder mit jedem Korb (100) verknüpft sind, um das Vorliegen eines auf die jeweilige Aufnahmebank (15) aufgeladenen Korbs (100) in dem Aufnahmezustand anzuzeigen, und wobei die visuellen Erfassungsmittel (36) auch imstande sind, um die Anzeige der visuellen Anzeigemittel (40) zu erfassen, um für die zentrale Steuereinheit (37) verwendbare Informationen zu erlangen.

2. Behandlungsanlage (10) nach Anspruch 1, **dadurch gekennzeichnet, dass** die visuellen Erfassungsmittel (36), die mindestens eine Videokamera (39) aufweisen, so konfiguriert sind, dass sie auch die Behandlungsmaschinen (16, 17, 18, 19, 20) rahmenerfassen, um Informationen über deren Verfügbarkeitsstatus zu erlangen, die für die zentrale Steuereinheit (37) verwendbar sind.

3. Behandlungsanlage (10) nach Anspruch 1, **dadurch gekennzeichnet, dass** die Anzeigemittel (40) Statusleuchtelemente (41) sind, die mit den Aufnahmebänken (15) verknüpft sind und die so konfiguriert sind, dass sie selektiv eine Lichtstrahlung aussenden, die imstande ist, den Aufnahmezustand eines korrespondierenden Korbs (100) anzuzeigen.

4. Behandlungsanlage (10) nach Anspruch 1, **dadurch gekennzeichnet, dass** die Anzeigemittel (40) Codierungselemente (101) sind, die mit jeweiligen Körben (100) verknüpft sind und die von den visuellen Erfassungsmitteln (36) gelesen und/oder erfasst werden können, um eine Anzeige des Aufnahmezustand eines korrespondierenden Korbs (100) zu geben.

5. Verfahren zum Behandeln von Gegenständen (G) mittels einer Anlage (10) nach den Ansprüchen von 1 bis 4, aufweisend einen Vorbehandlungsschritt, in dem die Gegenstände (G) in einer oder mehreren der Vorbehandlungsstationen (11, 12, 13) vorbehandelt werden, einen Positionierungsschritt, in dem die vorbehandelten Gegenstände (G) in jeweiligen Körben (100) positioniert werden, die auf den Aufnahmebänken (15) geeignet angeordnet sind, einen Bewegungsschritt, in dem mindestens ein Shuttle (21) mittels der ersten Steuervorrichtung (29), die empfangene sich auf den Weg beziehende Bewegungsinformationen ausführt, sich zwischen den Aufnahmebänken (15) und jeweiligen Einlassöffnungen (26) der Behandlungsmaschinen (16, 17, 18, 19, 20) bewegt, und einen Behandlungsschritt, in dem die Gegenstände (G) in einer der Behandlungsmaschinen (16, 17, 18, 19, 20) behandelt werden, **dadurch gekennzeichnet, dass** es einen Steuerungs- und Überwachungsschritt aufweist, in dem in einem Erfassungs-Teilschritt die visuellen Erfassungsmittel (36) mindestens die Aufnahmebänke (15) rahmenerfassen, um visuelle Informationen über einen Aufnahmezustand des mit vorbehandelten Gegenständen (G) versehenen Korbs (100) zu erlangen, der auf den Aufnahmebänken (15) in einer bestimmten Aufnahmeposition positioniert ist, und in dem in einem Verarbeitungs-Teilschritt die zentrale Steuereinheit (37) sowohl die visuellen Informationen als auch Informationen über den Verfügbarkeitsstatus der Behandlungsmaschinen (16, 17, 18, 19, 20) empfängt und verarbeitet, um die Bewegungsinformationen zu erzeugen, um die Bewegung des mindestens einen Shuttles (21) zu steuern, wobei die erste Steuervorrichtung (29) zur Kommunikation mit mindestens der zentralen Steuereinheit (37) mit einer Kommunikationsschnittstelle versehen ist und die Behandlungsmaschinen (16, 17, 18, 19, 20) jeweils zweite Steuervorrichtungen (30), die mit jeweiligen Kommunikationsschnittstellen versehen sind, aufweisen, wobei visuelle Anzeigemittel (40) mit den Aufnahmebänken (15) und/oder mit jedem Korb (100) verknüpft sind, um das Vorliegen eines auf die jeweilige Aufnahmebank (15) geladenen Korbs (100) im Aufnahmezustand anzuzeigen, und wobei die visuellen Erfassungsmittel (36) auch imstande sind, die Anzeige der visuellen Anzeigemittel (40) zu erfassen, um für die zentrale Steuereinheit (37) verwendbare Informationen zu erlangen.

6. Verfahren nach Anspruch 5, **dadurch gekennzeichnet, dass** in dem Erfassungs-Teilschritt die visuellen Erfassungsmittel (36) auch die Behandlungsmaschinen (16, 17, 18, 19, 20) rahmenerfassen, um Informationen über deren Verfügbarkeitsstatus zu erlangen, diese für die zentrale Steuereinheit (37) verwendbar machend.

7. Behandlungszentrum (200) zur Durchführung der vollständigen Behandlung von Vorwäsche, Wäsche, thermischer Desinfektion und Sterilisation von Gegenständen (G), das mindestens einen ersten, septischen Bereich (201), einen zweiten, sauberen Bereich (202) und einen dritten, sterilen Bereich (203) aufweist, die durch Trennmittel (205) voneinander getrennt sind, wobei der erste Bereich (201) durch eine Anlage (10) gemäß den Ansprüchen von 1 bis 4 definiert ist, wobei die Vorbehandlungsstationen (11, 12, 13) Stationen zum Vorwaschen der Gegenstände (G) sind und die Behandlungsmaschinen (16, 17, 18, 19, 20) Maschinen sind zum Waschen und thermischen Desinfizieren der bereits vorgewaschenen Gegenstände (G), und wobei der zweite Bereich (202) durch eine weitere Anlage (10) wie in den Ansprüchen von 1 bis 4 definiert ist, wobei die Vorbehandlungsstationen (11, 12, 13) Stationen zum Verpacken der gewaschenen und thermisch desinfizierten Gegenstände (G) sind und die Behandlungsmaschinen (16, 17, 18, 19, 20) Maschinen zum Sterilisieren der Gegenstände (G) sind, um sie für eine anschließende Lagerung in dem dritten Bereich (203) vorzubereiten.

## Revendications

1. Installation (10) de traitement d'objets (G) comprenant une pluralité de postes de prétraitement (11, 12, 13), une pluralité de bancs de ramassage (15) configurés pour ramasser des paniers (100) contenant des objets prétraités (G) et positionnés à proximité desdits postes de prétraitement (11, 12, 13), une pluralité de machines de traitement (16, 17, 18, 19, 20), et au moins une navette sans pilote mobile (21) pourvue à bord d'un premier dispositif de commande (29) pour recevoir et exécuter des informations de mouvement relatives à au moins un trajet entre lesdits bancs de ramassage (15) et des ouvertures d'entrée respectives (26) desdites machines de traitement (16, 17, 18, 19, 20), **caractérisé en ce qu'il** comprend un système de supervision (35) muni de moyens d'acquisition visuelle (36) connectés à une unité de commande centrale (37) qui communique au moins avec ledit premier dispositif de commande (29), lesdits moyens d'acquisition visuelle (36) étant configurés pour encadrer au moins lesdits bancs de ramassage (15) afin d'acquérir des informations visuelles sur une condition de ramassage d'un panier (100) muni d'objets prétraités (G) qui est présent sur l'un desdits des bancs de saisie (15), dans lesquels ladite unité de commande centrale (37) est configurée pour recevoir et traiter à la fois lesdites informations visuelles et également des informations sur l'état de disponibilité desdites machines de traitement (16, 17, 18, 19, 20), pour générer lesdites informations de mouvement afin de commander ledit mouvement de ladite au moins une navette (21), dans lesquels afin de communiquer avec au moins ladite unité de commande centrale (37), ledit premier dispositif de commande (29) est pourvu d'une interface de communication et lesdites machines de traitement (16, 17, 18, 19, 20) comprennent chacune des seconds dispositifs de commande (30) pourvus d'interfaces de communication respectives, dans lesquels des moyens d'indication visuelle (40) sont associés auxdits bancs de saisie (15) et/ou à chaque panier (100), pour indiquer la présence d'un panier (100), chargé sur le banc de saisie respectif (15), dans ladite condition de saisie, et dans lesquels lesdits moyens d'acquisition visuelle (36) sont également en mesure de détecter l'indication desdits moyens d'indication visuelle (40) pour obtenir des informations utilisables pour ladite unité de commande centrale (37).

2. Installation de traitement (10) selon la revendication 1, **caractérisée en ce que** lesdits moyens d'acquisition visuelle (36), comprenant au moins une caméra vidéo (39), sont configurés pour encadrer également lesdites machines de traitement (16, 17, 18, 19, 20) afin d'acquérir des informations sur l'état de disponibilité de celles-ci utilisables pour ladite unité de commande centrale (37).

3. Installation de traitement (10) selon la revendication 1, **caractérisée en ce que** lesdits moyens d'indication (40) sont des éléments de voyant d'état (41) associés auxdits bancs de ramassage (15) et configurés pour émettre sélectivement un rayonnement lumineux capable d'indiquer ledit état de ramassage d'un panier correspondant (100).

4. Installation de traitement (10) selon la revendication 1, **caractérisée en ce que** lesdits moyens d'indication (40) sont des éléments de codage (101) qui sont associés à des paniers respectifs (100), et qui sont lisibles et/ou détectables par lesdits moyens d'acquisition visuelle (36) afin de donner une indication de ladite condition de ramassage d'un panier correspondant (100).

5. Procédé de traitement d'objets (G) au moyen d'une installation (10) selon les revendications 1 à 4, comprenant une étape de prétraitement, dans laquelle lesdits objets (G) sont prétraités dans une ou plusieurs desdites stations de prétraitement (11, 12, 13), une étape de positionnement, dans laquelle lesdits objets prétraités (G) sont positionnés dans des paniers respectifs (100) disposés de manière appropriée sur lesdits bancs de ramassage (15), une étape de déplacement, dans laquelle au moins une navette (21), au moyen dudit premier dispositif de commande (29) qui exécute le déplacement reçu des informations relatives audit trajet, se déplace entre lesdits bancs de ramassage (15) et les ouvertures d'entrée respectives (26) desdites machines de traitement (16, 17, 18, 19, 20), et une étape de traitement, dans laquelle lesdits objets (G) sont traités dans l'une desdites machines de traitement (16, 17, 18, 19, 20), **caractérisé en ce qu'il** comprend une étape de commande et de supervision dans laquelle, dans une sous-étape d'acquisition, lesdits moyens d'acquisition visuelle (36) encadrent au moins lesdits bancs de ramassage (15) pour acquérir des informations visuelles sur une condition de ramassage dudit panier (100) pourvue d'objets prétraités (G) qui est positionnée sur lesdits bancs de ramassage (15) dans une certaine position de ramassage, et dans laquelle, dans une sous-étape de traitement, ladite unité de commande centrale (37) reçoit et traite à la fois lesdites informations visuelles et également des informations sur l'état de disponibilité desdites machines de traitement (16, 17, 18, 19, 20), pour générer lesdites informations de mouvement afin de commander ledit mouvement de ladite au moins une navette (21), dans lequel, afin de communiquer avec au moins ladite unité de commande centrale (37), ledit premier dispositif de commande (29) est muni d'une interface de communication et lesdites machines de traitement (16, 17, 18, 19, 20) comprennent chacune des seconds dispositifs de commande (30) munis d'interfaces de communication respectives, dans lesquels des moyens d'indication visuelle (40) sont associés auxdits bancs de ramassage (15) et/ou à chaque panier (100), pour indiquer la présence d'un panier (100), chargé sur le banc de ramassage respectif (15), dans ledit état de ramassage, et dans lesquels lesdits moyens d'acquisition visuelle (36) sont également en mesure de détecter l'indication desdits moyens d'indication visuelle (40) pour obtenir des informations utilisables pour ladite unité de commande centrale (37).

6. Procédé selon la revendication 5, **caractérisé en ce que** dans ladite sous-étape d'acquisition, lesdits moyens d'acquisition visuelle (36) encadrent également lesdites machines de traitement (16, 17, 18, 19, 20) pour acquérir des informations sur leur état de disponibilité, ce qui les rend utilisables pour ladite unité de commande centrale (37).

7. Centre de traitement (200) pour effectuer le traitement complet du prélavage, du lavage, de la désinfection thermique et de la stérilisation d'objets (G), qui comprend au moins une première zone septique (201), une deuxième zone propre (202) et une troisième zone stérile (203), qui sont séparées les unes des autres par des moyens de séparation (205), dans lequel ladite première zone (201) est définie par une installation (10) selon les revendications 1 à 4, dans lequel lesdits postes de pré-traitement (11, 12, 13) sont des postes de prélavage desdits objets (G) et lesdites machines de traitement (16, 17, 18, 19, 20) sont des machines de lavage et de désinfection thermique desdits objets déjà prélavés (G), et dans lequel ladite deuxième zone (202) est définie par une autre installation (10) selon les revendications 1 à 4, dans lequel lesdits postes de pré-traitement (11, 12, 13) sont des postes d'emballage desdits objets lavés et thermodésinfectés (G) et lesdites machines de traitement (16, 17, 18, 19, 20) sont des machines de stérilisation desdits objets (G), afin de les préparer pour un stockage ultérieur dans ladite troisième zone (203).
